# EUROPÄISCHE PATENTSCHRIFT

(11) **EP 0 855 380 B1**
(45) Veröffentlichungstag und Bekanntmachung des Hinweises auf die Patenterteilung: **17.04.2002**
(21) Anmeldenummer: 98100253.8
(22) Anmeldetag: 09.01.1998
(51) Int. Cl.: C07C 51/567, C07C 55/10

(54) **Verfahren zur Herstellung von Bernsteinsäureanhydrid**
Process for the preparation of succinic anhydride
Procédé pour la préparation d'anhydride succinique

(30) Priorität: 22.01.1997 DE 19702039
(43) Veröffentlichungstag der Anmeldung: 29.07.1998
(73) Patentinhaber: BAYER AG, 51368 Leverkusen (DE)
(72) Erfinder: Darsow, Gerhard, Dr., 47804 Krefeld (DE)

(56) Entgegenhaltungen:
- EP-A- 0 722 924

## Beschreibung

Die vorliegende Erfindung betrifft ein kostengünstiges, kontinuierlich mit besonders hohen Umsätzen arbeitendes Verfahren zur Herstellung von Bernsteinsäureanhydrid, bei welchem nur äußerst geringe Mengen des üblicherweise bei der Hydrierung von Maleinsäureanhydrid als Nebenprodukt entstehenden γ-Butyrolactons sowie keine Mono- und keine Hydroxycarbonsäuren der C-Zahlen < 4 gebildet werden.

Bernsteinsäureanhydrid ist ein wichtiges Ausgangsprodukt für die Herstellung von thermoplastischen Polyestern, die besondere mechanische und chemische Eigenschaften sowie eine gute biologische Abbaubarkeit aufweisen.

Es ist bekannt, Bernsteinsäureanhydrid aus Bernsteinsäure diskontinuierlich durch eine Dehydratisierungsreaktion durch Einleiten von Essigsäureanhydrid-Dämpfen in geschmolzene Bernsteinsäure herzustellen (GB 507 592). Es ist ferner bekannt, Maleinsäureanhydrid im Batch-Prozeß über Ni (US 2 198 153, zitiert nach Chem. Abstracts 34 (1940), 5465⁹) oder Pd, Rh, Pt/Al₂O₃ (JP 48-7609 (1973)) zu Bernsteinsäureanhydrid zu hydrieren. Es ist weiterhin bekannt, Maleinsäureanhydrid über Pd/A-Kohle kontinuierlich zu Bernsteinsäureanhydrid zu hydrieren (SU 721 406; zitiert nach Chem. Abstacts 93 (1980), 71047 b). Außerdem ist bekannt, Maleinsäureanhydrid über Katalysatoren aus Cu-, Co- und bzw. oder Ni-molybdat, -wolframat, - chromat und bzw. oder -vanadat, wozu als Träger geformte Kieselsäurestränge eingesetzt werden, zu Bernsteinsäureanhydrid zu hydrieren (DE 1 226 556).

Der Reaktionsverlauf läßt sich durch das folgende Reaktionsschema veranschaulichen:

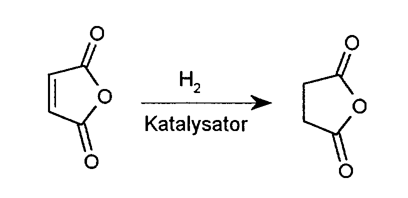

Bei den bekannten Verfahren zur Herstellung von Bernsteinsäureanhydrid werden überwiegend diskontinuierliche Suspensionsverfahren (Batch-Prozesse) angewendet, bei denen das Maleinsäureanhydrid mit und ohne Lösungsmittel über pulverförmigen Katalysatoren mit Wasserstoff hydriert wird. Diskontinuierliche Verfahren haben den Nachteil, daß ihre Kapazität relativ zum Reaktionsvolumen sehr klein ist und somit ein Bedarf nach großen Reaktionsapparaturen und Lagertanks besteht. Energieverbrauch und Personalbedarf sind verhältnismäßig hoch.

Kontinuierliche Pulverkatalysatorverfahren, die mit mehreren in Kaskade geschalteten Hydrierreaktoren arbeiten, vermeiden einen Teil dieser Nachteile. Es bleibt jedoch das Erfordernis, die pulverförmigen Katalysatoren mehrfach gezielt zu dosieren, umzupumpen und quantitativ vom Reaktionsprodukt abzufiltrieren. Die Katalysatorschlammpumpen unterliegen einem hohen mechanischen Verschleiß. Die quantitative Entfernung der pulverförmigen Katalysatoren aus dem Reaktionsprodukt ist aufwendig. Ferner ist die Gefahr groß, die Katalysatoraktivität durch die zusätzlichen Operationen verhältnismäßig schnell zu verringern. Es ist daher vorteilhaft, die Reaktion über fest angeordneten Katalysatoren ablaufen zu lassen. Solche Katalysatoren müssen eine hohe Aktivität besitzen, die über einen längeren Zeitraum nicht nachlassen darf, weil häufige Katalysatorwechsel bei Festbettreaktionen ebenfalls aufwendig sind. Als kontinuierlich arbeitendes Verfahren wurde bisher die Hydrierung von Maleinsäureanhydrid zu Bernsteinsäureanhydrid über Pd/A-Kohle bzw. Cu-, CO- und bzw. oder Ni-molybdat, -wolframat, -chromat und bzw. -vanadat auf Kieselsäureträger beschrieben (DE 1 226 556). Diese Katalysatoren hatten nur eine begrenzte Lebensdauer. Außerdem kann die Reaktion in der Regel nur mit Hilfe eines Lösungsmittels durchgeführt werden.

Ferner wurde gefunden, daß man Maleinsäureanhydrid über im Festbett angeordneten trägerfreien Formkörpern aus sauerstofffreien Metallpulvern von einem oder mehreren Elementen der Eisenuntergruppe der VIII. Nebengruppe des Periodensystems der Elemente (Mendelejew) zu Bernsteinsäureanhydrid hydrieren kann, wozu es nützlich sein kann, die Metalle der Eisenuntergruppe mit aktivierend wirkenden Elementen der VI. Nebengruppe des Periodensystems zu legieren. Dabei können die zur Herstellung der Formkörper eingesetzten Pulver zusätzlich kleine Anteile nicht katalytisch wirkender Elemente (z.B. Silizium, Aluminium, Titan, Kohlenstoff) enthalten (EP 722 924 A). Die Umsätze sind jedoch mit Katalysatorbelastungen von 0,14-0,4 kg Maleinsäureanhydrid pro 1 Katalysator · h sehr niedrig.

Überraschenderweise wurde nun gefunden, daß man Maleinsäureanhydrid mit erheblich höheren Umsätzen (z.B. 0,6 - 1,4 kg Maleinsäureanhydrid pro l Katalysator h) sehr gut kontinuierlich über im Festbett angeordneten trägerfreien Formkörpern aus sauerstofffreien Metallpulvern von einem oder mehreren Elementen der Eisenuntergruppe der VIII. Nebengruppe des Periodensystems der Elemente (Mendelejew), die zusätzlich mit einem oder mehreren aktivierend wirkenden Elementen der IV. und/oder V. Nebengruppe des Periodensystems legiert sind, zu Bernsteinsäureanhydrid hydrieren kann. Dabei können die zum Einsatz kommenden Pulver zusätzlich gewisse Anteile nicht katalytisch wirkender Elemente (z.B. Silizium, Aluminium, Kohlenstoff) enthalten, ohne daß die hohe Aktivität gemindert wird. Die Festkörper müssen eine Druckfestigkeit von 20-220 N und eine innere Oberfläche von 10-100 m²/g aufweisen.

Gegenstand der Erfindung ist damit ein Verfahren zur kontinuierlichen Herstellung von Bernsteinsäureanhydrid durch katalysierte Hydrierung von Maleinsäureanhydrid, das dadurch gekennzeichnet ist, daß die Hydrierung in der flüssigen Phase bei einem H₂-Druck von 10 - 400 bar, einer 10-80-fachen molaren H₂-Menge, bezogen auf die stöchiometrische Menge, und bei einer Temperatur von 60-180°C an im Festbett angeordneten sauerstofffreien und trägerfreien Katalysatoren durchgeführt wird, wobei die Katalysatoren als verpreßte, aus Metallpulvern hergestellte Formkörper vorliegen, die eine Druckfestigkeit von 20-220 N und eine innere Oberfläche von 10-100 m²/g aufweisen und bei denen die Metallpulver mindestens 50 Gew.-% eines oder mehrerer Elemente der Eisengruppe der VIII. Nebengruppe des Periodensystems der Elemente (Mendelejew), vermischt oder legiert mit mindestens 6 Gew.-% eines oder mehrerer Metalle der IV. und/oder V. Nebengruppe des Periodensystems und 0 bis 20 Gew.-% eines oder mehrerer hydrierinerter Elemente aus der Gruppe von Aluminium, Silicium und Kohlenstoff, alles bezogen auf das Gesamtgewicht des Metallpulvers, enthalten.

Die Druckfestigkeit der trägerfreien Formkörper kann nach DIN 50106 bestimmt werden. Die Überprüfung von trägerfreien Formkörpern auf die anspruchsgemäßen inneren Oberflächen und damit auf Brauchbarkeit für das erfindungsgemäße Verfahren kann nach Methoden durchgeführt werden, die von F. M. Nelsen und F.T. Eggertsen, Analyt. Chem. 30 (1958), S. 1387-1390 bzw. S. J. Gregg und K.S.W. Sing, Adsorption, Surface Area and Porosity, London 1982, Kap. 2 und 6, beschrieben sind.

Die Eisenuntergruppe der VIII. Nebengruppe des Periodensystems der Elemente (Mendelejew) enthält die Elemente Eisen, Kobalt und Nickel. Die erfindungsgemäß zu verwendenden trägerfreien Formkörper enthalten eines oder mehrere dieser Metalle in Mengen von mindestens 50, vorzugsweise mindestens 60, insbesondere mindestens 65 Gew.-%, bezogen auf das Gesamtgewicht der trägerfreien Formkörper.

Die IV. Nebengruppe des Periodensystems enthält die Elemente Titan, Zirkon und Hafnium. Die V. Nebengruppe des Periodensystems enthält die Elemente Vanadium, Niob und Tantal. Die erfindungsgemäß zu verwendenden trägerfreien Formkörper enthalten eines oder mehrerer dieser Metalle in Mengen von mindestens 6,0 Gew.-%, vorzugsweise mindestens 9,5 Gew.-%, bezogen auf trägerfreie Formkörper; sie enthalten eines oder mehrere dieser Metalle in Mengen von höchstens 30, vorzugsweise höchstens 15 Gew.-%, bezogen auf trägerfreie Formkörper. Bevorzugt unter den genannten Elementen sind Zirkon, Hafnium, Vanadium Niob und Tantal, besonders bevorzugt Zirkon und Vanadium.

Die erfindungsgemäß zu verwendenden trägerfreien Formkörper können darüber hinaus, jeweils bezogen auf trägerfreie Formkörper, bis zu 20, vorzugsweise bis zu 15 Gew.-% anderer Elemente enthalten; Beispiele solcher Elemente, die nicht katalytisch wirken, umfassen Aluminium, Silicum und Kohlenstoff. Nach einer bevorzugten Ausführungsform enthalten die trägerfreien Formkörper außer den Metallen der VIII. und IV. und/oder V. Nebengruppe nicht mehr als 15 Gew.-% Aluminium und nicht mehr als 5 Gew.-% anderer Elemente.

Für den Hydrierprozeß wird ein auf einen Druck von 10 - 400 bar, bevorzugt 30 bis 400 bar, besonders bevorzugt 50-300 bar, vorkomprimierter reiner Wasserstoff eingesetzt, wobei man mit 10 - 80-fachen, bevorzugt 20- bis 40-fachen molaren Wasserstoffmengen, bezogen auf die stöchiometrische Menge, arbeitet.

Die Hydrierung erfolgt kontinuierlich im Festbettverfahren an den als Hydrierungskatalysatoren dienenden trägerfreien Formkörpern der beschriebenen Art, indem man das zu hydrierende flüssige Maleinsäureanhydrid entweder im Gleichstrom mit dem zuvor zugemischten Wasserstoff von unten nach oben aufsteigend über die in den Hydrierreaktor gefüllten Formkörper strömen läßt oder auch dem von oben einströmenden Wasserstoff von unten kommend entgegenführt (Gegenstromverfahren). Das erfindungsgemäße Verfahren kann selbstverständlich auch in Lösungsmitteln durchgeführt werden. Geeignete, gegebenenfalls mitzuverwendende und unter den Reaktionsbedingungen inerte Lösungsmittel sind beispielsweise Di-n-propyl-ether, Diiso-propylether, Di-n-butyl-ether, Di-iso-butyl-ether, Tetrahydrofuran, Dioxan, γ-Butyrolacton. In bevorzugter Weise wird das erfindungsgemäße Verfahren ohne Lösungsmittel oder in γ-Butyrolacton als systemeigenem Lösungsmittel durchgeführt.

Der Hydrierprozeß wird bei Temperaturen von 60 bis 180°C, vorzugsweise 80-160°C, besonders bevorzugt 100-140°C, durchgeführt. Niedrigere Temperaturen bedingen höhere Verweilzeiten oder den Verzicht auf einen quantitativen Umsatz. Höhere Temperaturen führen zu vermehrter Bildung von γ-Butyrolacton als Nebenprodukt.

Die stündliche Katalysatorbelastung beträgt 600 bis 1400 g Maleinsäureanhydrid/l Katalysator.

Zum Einsatz kommt Maleinsäureanhydrid im allgemeinen in der technisch verfügbaren Qualität, bevorzugt mit einer Reinheit > 99 %. Es lassen sich aber auch Destillationsrückläufe, z.B. von γ-Butyrolacton, mit darin enthaltenem Maleinsäureanhydrid einsetzen.

Der Hydrierreaktor kann entweder ein einzelnes Hochdruckrohr aus Stahl oder einer Stahllegierung sein, das mit den trägerfreien Formkörpern ganz oder teilweise gefüllt wird, wobei auch die Anwendung auf Horden (Drahtkörbe u.ä.) nützlich sein kann, oder aber ein ummanteltes Hochdruckrohrbündel, dessen Einzelrohre mit Formkörpern ganz oder teilweise gefüllt werden.

Die Herstellung der trägerfreien Formkörper kann nach gebräuchlichen Methoden durch Verpressen der Metallpulver auf Tablettier- oder Pelletiermaschinen unter hohem Druck erfolgen, wobei zur Verbesserung des Haftvermögens der Metallpartikel auch Graphit in Mengen von 0,5 -1,5 Gew.-%, bezogen auf das Gesamtgewicht der den Katalysator bildenden Bestandteile, oder Klebstoffe in kleinen Mengen zum Einsatz kommen können. Die Herstellung der trägerfreien Formkörper erfolgt vorzugsweise in einer sauerstofffreien Atmosphäre, um Oberflächenoxidationen zu vermeiden. Am wirksamsten und für die Reaktionsführung am günstigsten sind tablettierte oder pelletierte Formkörper mit Durchmessern von 2-10 mm, bevorzugt von 3 bis 7 mm. Von erheblicher Bedeutung ist die Druckfestigkeit der Formkörper, die erfindungsgemäß bei Werten von 20 bis 220 N, bevorzugt 60 bis 200 N, liegt. Niedrigere Druckfestigkeiten führen zu Formkörperzerfall bzw. erosivem Abrieb, was eine metallische Kontamierung des Reaktionsproduktes bewirken würde. Höhere Werte bedingen einen unangemessenen Aufwand beim Verpressen, ohne daß weitere Vorteile erzielt werden. Von erheblicher Bedeutung ist weiterhin die innere Oberfläche der Formkörper, die erfindungsgemäß bei Werten von 10 bis 100 m²/g liegt und ausschlaggebend für einen möglichst quantitativen Umsatz der Einsatzstoffe ist. Unter den geschilderten Reaktionsbedingungen sind auf diese Weise ganz unerwartet hohe Katalysatorstandzeiten von 15.000 Stunden und mehr zu erzielen, was zu Katalysatorverbräuchen < 0,1 Gew.-%, bezogen auf das hergestellte Reaktionsprodukt, führt. Die erfindungsgemäß einzusetzenden sauerstofffreien und trägerfreien Festbettkatalysatoren neigen im Gegensatz zu trägerhaltigen Katalysatoren nicht zum "Ausbluten", d.h. nicht zum Übergang von Katalysatorbestandteilen in ionischer oder kolloidaler Form in die Lösungsphase des Substrats, so daß das Substrat nicht durch Schwermetalle kontaminiert wird, die normalerweise nur mühsam, beispielsweise mit Hilfe von Ionenaustauschern, aus dem Substrat entfernt werden können. Die einzusetzenden Katalysatormetalle können, etwa nach längerem Gebrauch des Katalysators, leicht aufgearbeitet und wiederverwendet werden, da die Schwermertalle nicht umständlich von einem Trägermaterial getrennt werden müssen.

Das den Hydrierreaktor verlassende Reaktionsgemisch wird entspannt, wobei man den überschüssigen Wasserstoff abfangen und nach erfolgter Komprimierung und Ergänzung von verbrauchtem Wasserstoff erneut zum Einsatz bringen kann. Bei einer kompletten Hydrierung (99,9 bis 100 %iger Umsatz des Maleinsäureanhydrids) besteht das Reaktionsgemisch zu mindestens 98 Gew.-% aus Bernsteinsäureanhydrid. Es kann an organischen Leichtsiedern bis zu 1,6 Gew.-%, hauptsächlich als γ-Butyrolacton, enthalten. Das erzeugte Bernsteinsäureanhydrid weist einen Gehalt an Katalysatorbestandteilen von < 1 ppm auf, wird nach der destillativen Entfernung der Leichtsieder in einer Reinheit von ≥ 99,9 Gew.-% erhalten und ist daher ohne jede weitere Reinigung zur weiteren Verwendung, beispielsweise auch zur Herstellung von Polymeren einsetzbar. Die nach der Destillation erhaltene farblose und glasklare Schmelze von Bernsteinsäureanhydrid kann entweder in Kristallisationsgeräten üblicher Bauart zur Kristallisation gebracht werden oder über Abschuppwalzen zu rieselfähigen Schuppen verarbeitet werden.

### Beispiele

### Beispiel 1

Ein senkrecht stehendes, wärmeisoliertes Hochdruckrohr aus nichtrostendem Stahl von 45 mm Innendurchmesser und 1 m Länge wurde mit 1,21 eines durch Tablettierung eines Metallpulvers aus einer Ni-/Zr-/Al-Legierung mit einem Zr-Gehalt von 14,9 Gew.-% und einem Al-Gehalt von 10,5 Gew.-% hergestellten Katalysators gefüllt, der bei einer Zylinderhöhe von 5 mm und einem Durchmesser von 5 mm eine Druckfestigkeit von 78 N auf die Zylindermantelfläche und eine innere Oberfläche von 81 m²/g aufwies. Durch dieses Rohr wurden stündlich 700 g einer in einem Schmelzkessel hergestellten glasklaren Schmelze von Maleinsäureanhydrid gemeinsam mit der 20fachen molaren Menge von unter einem Druck von 300 bar stehendem, hochreinem Wasserstoff kontinuierlich von unten nach oben aufsteigend gepumpt. Maleinsäureanhydridschmelze und Wasserstoff wurden vorab gemeinsam durch einen Wärmeaustauscher geführt und so hoch erhitzt, daß sie in das Hochdruckrohr mit einer Temperatur von 125°C eintraten. Das das Hochdruckrohr verlassende Gemisch aus flüssigem Reaktionsprodukt und überschüssigem Wasserstoff wurde in einen Abscheider geführt, von wo der Wasserstoff nach Ersatz der verbrauchten Menge wieder zusammen mit neuer Maleinsäureanhydridschmelze in den Vorwärmer und von da erneut in das Hochdruckrohr gepumpt wurde.

Die farblose und klare Schmelze des Reaktionsproduktes wurde nach Entspannung auf Normaldruck und Abkühlung gaschromatographisch untersucht. Sie enthielt kein Maleinsäureanhydrid mehr und an organischen Leichtsiedern nur 1,2 Gew.-% γ-Butyrolacton, so daß der Bernsteinsäureanhydridgehalt des Reaktionsproduktes bei 98,8 Gew.-% lag. Das erzeugte Bernsteinsäureanhydrid wurde nach der destillativen Entfernung des Leichtsieders in einer Reinheit von 99,9 Gew.-% erhalten.

Der Katalysator war nach einer Laufzeit von 3.600 Stunden unverändert wirksam, so daß sich die Zusammensetzung des Reaktionsproduktes über diesen Zeitraum nicht veränderte.

### Beispiel 2

In einem Hochdruckrohr wie in Beispiel 1 wurde bei einer Temperatur von 125°C und einem Wasserstoffdruck von 200 bar der Wasserstoff im ungekehrten Reaktionsfluß wie in Beispiel 1 der aufsteigenden Maleinsäureanhydridschmelze entgegengeführt, wobei stündlich eine gleich große Menge wie in Beispiel 1 hydriert wurde. Der Katalysator war durch Tablettierung einer pulverisierten Ni-/Fe-/Zr-Legierung gewonnen worden. Die Legierung enthielt einen Fe-Anteil in Nickel von 5,4 Gew.-% und einen Zr-Anteil von 10,9 Gew.-%. Die Tabletten hatten bei einer Zylinderhöhe von 5 mm und einem Durchmesser von 5 mm eine Druckfestigkeit von 107 N auf die Zylindermantelfläche und eine innere Oberfläche von 93 m²/g. Nach einer Laufzeit von 1.200 Stunden lag der Umsatz des eingesetzten Maleinsäureanhydrids bei 99,9 Gew.-%. Der Gehalt an γ-Butyrolacton im Reaktionsprodukt lag bei 1,6 Gew.-%, so daß der Bernsteinsäureanhydridgehalt des Reaktionsproduktes bei 98,3 Gew.-% lag (Rest = 0,1 Gew.-% nicht umgesetztes Maleinsäureanhydrid). Nach der destillativen Entfernung der Verunreinigungen wurde das erzeugte Bernsteinsäureanhydrid in einer Reinheit von 99,9°Gew.-% erhalten.

### Beispiel 3

Ein senkrecht stehendes, wärmeisoliertes Hochdruckrohr aus nichtrostendem Stahl von 45 mm Innendurchmesser und 1 m Länge wurde mit 1,2 l eines durch Tablettierung von Pulver einer Ni-/Zr-/V-/Al-Legierung hergestellten Hydrierungskatalysators gefüllt, der bei einer Zylinderhöhe von 5 mm und einem Durchmesser von 5 mm eine Druckfestigkeit von 106 N auf die Zylindermantelfläche und eine innere Oberfläche von 81 m²/g aufwies. Die Legierung enthielt einen Zr-Anteil von 14,9 Gew.-%, einen V-Anteil von 12,1 Gew.-% und einen Al-Anteil von 10,2 Gew.-%. Durch das Hochdruckrohr wurden stündlich 800 g Maleinsäureanhydridschmelze gemeinsam mit der dreißigfachen molaren Menge von unter einem Druck von 300 bar stehendem hochreinem Wasserstoff von unten nach oben aufsteigend gepumpt. Maleinsäureanhydridschmelze und Wasserstoff wurden vor Eintritt in das Hochdruckrohr auf eine Temperatur von 120°C gebracht. Nach einer Laufzeit von 1.280 Stunden lag der Umsatz des eingesetzten Maleinsäureanhydrids bei 100 Gew.-%. Der Gehalt an γ-Butyrolacton im Reaktionsprodukt lag bei 1,0 Gew.-%, so daß der Bernsteinsäureanhydridgehalt bei 99,0 Gew.-% lag. Nach der destillativen Entfernung der Verunreinigung wurde das gewonnene Bernsteinsäureanhydrid in einer Reinheit von 99,9 Gew.-% erhalten.

### Beispiel 4

In einem Hochdruckrohr wie in Beispiel 1, aber aus Hochdruckstahl N9, wurde bei einer Temperatur von 120°C und einem Wasserstoffdruck von 300 bar stündlich eine gleich große Menge Maleinsäureanhydrid hydriert. Der Katalysator wurde durch Tablettierung von Pulver einer Ni-/V-/Al-Legierung mit einem V-Gehalt von 12,1 Gew.-% und einem Al-Gehalt von 6,1 Gew.-% hergestellt. Die Tabletten hatten bei einer Zylinderhöhe von 5 mm und einem Durchmesser von 5 mm eine Druckfestigkeit von 97 N und eine innere Oberfläche von 85 m²/g. Nach einer Laufzeit von 800 Stunden lag der Gehalt an Bernsteinsäureanhydrid im Reaktionsprodukt bei 99,1 Gew.-% und der Gehalt an γ-Butyrolacton bei 0,9 Gew.-%.

### Beispiel 5

In einem Hochdruckrohr wie in Beispiel 1 wurde bei einer Temperatur von 130°C und einem Wasserstoffdruck von 300 bar stündlich eine Menge von 700 g Maleinsäureanhydrid hydriert. Der Katalysator wurde durch Tablettierung einer pulverisierten Ni-/Zr-Legierung mit einem Zr-Gehalt von 9,2 Gew.-% gewonnen. Die Tabletten hatten bei einer Zylinderhöhe von 5 mm und einem Durchmesser von 5 mm eine Druckfestigkeit von 118 N und eine innere Oberfläche von 98 m²/g. Nach einer Laufzeit von 400 Stunden lag der Umsatz des eingesetzten Maleinsäureanhydrids bei 99,9 Gew.-%. Der Gehalt an γ-Butyrolacton im Reaktionsprodukt betrug 1,0 Gew.-%, so daß der Bernsteinsäureanhydridgehalt bei 98,9 Gew.-% lag (Rest = 0,1 Gew.-% nicht umgesetztes Maleinsäureanhydrid).

### Beispiel 6

In einem Hochdruckrohr wie in Beispiel 1 wurde bei einer Temperatur von 120°C und einem Wasserstoffdruck von 300 bar stündlich eine Menge von 800 g Maleinsäureanhydrid, gelöst als 30 gew.-%ige Lösung in γ-Butyrolacton, hydriert. Der Katalysator wurde durch Tablettierung einer pulverisierten Ni-/Zr-/Al-/Si-Legierung mit einem Zr-Gehalt von 9,8 Gew.-%, einem Al-Gehalt von 6,1 Gew.-% und einem Si-Gehalt von 2,1 Gew.-% hergestellt. Die Tabletten hatten bei einer Zylinderhöhe von 5 mm und einem Durchmesser von 5 mm eine Druckfestigkeit von 116 N auf die Zylindermantelfläche und eine innere Oberfläche von 88 m²/g. Nach einer Laufzeit von 680 Stunden lag der Umsatz des eingesetzten Maleinsäureanhydrids bei 99,9 Gew.-%. Nach der destillativen Entfernung des Lösungsmittels und einer danach durchgeführten Destillation des rohen Bernsteinsäureanhydrids hatte dieses eine Reinheit von 99,95 Gew.-%. Das abdestillierte γ-Butyrolacton wurde wieder als Lösungsmittel in den Prozeß zurückgeführt.

## Patentansprüche

1. Verfahren zur kontinuierlichen Herstellung von Bernsteinsäureanhydrid durch katalytische Hydrierung von Maleinsäureanhydrid, **dadurch gekennzeichnet, daß** die Hydrierung in der flüssigen Phase bei einem H₂-Druck von 10 bis 400 bar, einer 10- bis 80-fachen molaren H₂-Menge, bezogen auf die stöchiometrische Menge, und bei einer Temperatur von 60 bis 180°C an im Festbett angeordneten sauerstofffreien und trägerfreien Katalysatoren durchgeführt wird, wobei die Katalysatoren als verpreßte, aus Metallpulvern hergestellte Formkörper vorliegen, die eine Druckfestigkeit von 20 bis 220 N und eine innere Oberfläche von 10 bis 95 m²/g aufweisen und bei denen die Metallpulver mindestens 50 Gew.-% eines oder mehrerer Elemente der Eisengruppe der VIII. Nebengruppe des Periodensystems der Elemente (Mendelejew), vermischt oder legiert mit mindestens 6 Gew.-% eines oder mehrerer Metalle der IV. und/oder V. Nebengruppe des Periodensystems und 0 bis 20 Gew.-% eines oder mehrerer hydrierinerter Elemente aus der Gruppe von Aluminium, Silicium und Kohlenstoff, alles bezogen auf das Gesamtgewicht des Metallpulvers, enthalten.

2. Verfahren nach Anspruch 1, **dadurch gekennzeichnet, daß** die Metallpulver mindestens 60 Gew.-%, insbesondere mindestens 65 Gew.% eines oder mehrerer Elemente der Eisengruppe enthalten.

3. Verfahren nach Anspruch 1, **dadurch gekennzeichnet, daß** die Metallpulver einen Gehalt von mindestens 7,5 Gew.%, insbesondere mindestens 9 Gew.-% an Elementen der IV. und/oder V. Nebengruppe enthalten.

4. Verfahren nach Anspruch 1, **dadurch gekennzeichnet, daß** die Metallpulver höchstens 30 Gew.-%, vorzugsweise höchstens 20 Gew.-% und insbesondere höchstens 15 Gew.-% an Elementen der IV. und/oder V. Nebengruppe enthalten.

5. Verfahren nach Anspruch 1, **dadurch gekennzeichnet, daß** die Metallpulver beim Vorliegen von hydrierinerten Elementen einen Gehalt von 0 bis 15 Gew.-% an Aluminium und von 0 bis 5 Gew.-% je Element Si und C enthalten.

6. Verfahren nach Anspruch 1, **dadurch gekennzeichnet, daß** die Formkörper eine Druckfestigkeit von 70 bis 140 N aufweisen.

7. Verfahren nach Anspruch 1, **dadurch gekennzeichnet, daß** die Formkörper zylinder- oder kugelförmig sind und Durchmesser von 2-10 mm, vorzugsweise 3 bis 7 mm besitzen.

8. Verfahren nach Anspruch 1, **dadurch gekennzeichnet, daß** die Hydrierung bei einem H₂-Druck von 30 bis 400 bar, bevorzugt 50-300 bar, durchgeführt wird.

9. Verfahren nach Anspruch 1, **dadurch gekennzeichnet, daß** bei einer 20- bis 40-fachen molaren H₂-Menge gearbeitet wird.

10. Verfahren nach Anspruch 1, **dadurch gekennzeichnet, daß** das Maleinsäureanhydrid den Hydrierreaktor von unten nach oben aufsteigend passiert, während der für die Hydrierung benötigte Wasserstoff entweder gemeinsam mit dem Maleinsäureanhydrid in den Reaktor gepumpt wird oder diesem, von oben nach unten strömend, entgegengeführt wird.

## Claims

1. Process for the continuous preparation of succinic anhydride by catalytic hydrogenation of maleic anhydride, **characterized in that** the hydrogenation is carried out in the liquid phase at an H₂ pressure of 10 to 400 bar, a 10 to 80 times molar amount of H₂, based on the stoichiometric amount, and at a temperature of 60 to 180°C and on oxygen-free support-free catalysts which are arranged in the fixed bed, where the catalysts are present as pressed shaped bodies which are produced from metal powders and have a compressive strength of 20 to 220 N and an internal surface area of 10 to 95 m²/g and in which the metal powders contain at least 50% by weight of one or more elements of the iron group of subgroup VIII of the Periodic Table of Elements (Mendeleer), mixed or alloyed with at least 6% by weight of one or more metals of subgroup IV and/or V of the Periodic Table, and 0 to 20 % by weight of one or more hydrogenation-inert elements from the group consisting of aluminium, silicon and carbon, all based on the total weight of metal powder.

2. Process according to Claim 1, **characterized in that** the metal powders contain at least 60 % by weight, in particular at least 65 % by weight, of one or more elements of the iron group.

3. Process according to Claim 1, **characterized in that** the metal powders have a content of at least 7.5 % by weight, in particular at least 9% by weight, of elements of subgroup IV and/or V.

4. Process according to Claim 1, **characterized in that** the metal powders contain at most 30% by weight, preferably at most 20% by weight, and in particular at most 15% by weight, of elements of subgroup IV and/or V.

5. Process according to Claim 1, **characterized in that** the metal powders, when hydrogenation-inert elements are present, have a content of 0 to 15 % by weight of aluminium and of 0 to 5 % by weight per element of Si and C.

6. Process according to Claim 1, **characterized in that** the shaped bodies have a compressive strength of 70 to 140 N.

7. Process according to Claim 1, **characterized in that** the shaped bodies are cylindrical or spherical and have diameters of 2-10 mm, preferably of 3 to 7 mm.

8. Process according to Claim 1, **characterized in that** the hydrogenation is carried out at an H₂ pressure of 30 to 400 bar, preferably 50-300 bar.

9. Process according to Claim 1, **characterized in that** a 20 to 40 times molar amount of H₂ is employed.

10. Process according to Claim 1, **characterized in that** the maleic anhydride passes through the hydrogenation reactor ascending from bottom to top, while the hydrogen required for the hydrogenation is either pumped into the reactor together with the maleic anhydride or is conducted in the opposite direction to this, flowing from top to bottom.

## Revendications

1. Procédé pour la préparation continue de l'anhydride succinique par hydrogénation catalytique de l'anhydride maléique, **caractérisé en ce que** l'on réalise l'hydrogénation en phase liquide, sous une pression d'hydrogène de 10 à 400 bar, avec une quantité 10 à 80 fois molaire d'hydrogène par rapport à la quantité théorique, et à une température de 60 à 180°C, sur des catalyseurs exempts d'oxygène et sans support, disposés en lit fixe, les catalyseurs étant à l'état de corps moulés par compression de poudres métalliques, ces corps ayant une résistance à la compression de 20 à 220 N et une surface interne de 10 à 95 m²/g, les poudres métalliques contenant au moins 50 % en poids d'un ou plusieurs éléments de la série du fer du sous-groupe VIII de la Classification Périodique des éléments (Mendelejew), mélangés ou alliés avec au moins 6 % en poids d'un ou plusieurs métaux du sous-groupe IV et/ou V de la Classification Périodique et 0 à 20 % en poids d'un ou plusieurs éléments, inertes à l'hydrogénation, du groupe de l'aluminium, du silicium et du carbone, tous ces pourcentages se rapportant au poids total de la poudre métallique.

2. Procédé selon la revendication 1, **caractérisé en ce que** les poudres métalliques contiennent au moins 60 % en poids, plus spécialement au moins 65 % en poids d'un ou plusieurs éléments de la série du fer.

3. Procédé selon la revendication 1, **caractérisé en ce que** les poudres métalliques contiennent au moins 7,5 % en poids, plus spécialement au moins 9 % en poids d'éléments des sous-groupes IV et/ou V.

4. Procédé selon la revendication 1, **caractérisé en ce que** les poudres métalliques contiennent au maximum 30 % en poids, de préférence au maximum 20 % en poids et plus spécialement au maximum 15 % en poids d'éléments des sous-groupes IV et/ou V.

5. Procédé selon la revendication 1, **caractérisé en ce que** les poudres métalliques, lorsqu'elles contiennent des éléments inertes à l'hydrogénation, contiennent de 0 à 15 % en poids d'aluminium et de 0 à 5 % en poids de chacun des éléments SI et C.

6. Procédé selon la revendication 1, **caractérisé en ce que** les corps moulés ont une résistance à la compression de 70 à 140 N.

7. Procédé selon la revendication 1, **caractérisé en ce que** les corps moulés ont une forme cylindrique ou sphérique avec des diamètres de 2 à 10 mm, de préférence de 3 à 7 mm.

8. Procédé selon la revendication 1, **caractérisé en ce que** l'hydrogénation est réalisée sous une pression d'hydrogène de 30 à 400 bar, de préférence de 50 à 300 bar.

9. Procédé selon la revendication 1, **caractérisé en ce que** l'on opère avec une quantité d'hydrogène 20 à 40 fois molaire.

10. Procédé selon la revendication 1, **caractérisé en ce que** l'anhydride maléique passe de bas en haut, en mouvement ascendant dans le réacteur d'hydrogénation, et l'hydrogène est pompé dans le réacteur avec l'anhydride maléique ou envoyé à contre-courant de celui-ci, de haut en bas.
